# EUROPÄISCHE PATENTSCHRIFT

(11) **EP 1 725 184 B1**
(45) Veröffentlichungstag und Bekanntmachung des Hinweises auf die Patenterteilung: **10.05.2017**
(21) Anmeldenummer: 04802924.3
(22) Anmeldetag: 11.12.2004
(51) Int. Cl.: A61F 2/91, A61F 2/915

(54) **STENT**
STENT
STENT

(30) Priorität: 16.03.2004 DE 102004012981
(43) Veröffentlichungstag der Anmeldung: 29.11.2006
(73) Patentinhaber: Merit Medical Systems, Inc., South Jordan, UT 84095 (US)
(72) Erfinder: NISSL, Thomas, 21441 Garstedt (DE)
(74) Vertreter: Griepenstroh, Jörg
(86) Internationale Anmeldenummer: PCT/DE2004/002719
(87) Internationale Veröffentlichungsnummer: WO 2005/089672

(56) Entgegenhaltungen:
- WO-A-02/100298
- WO-A1-2005/046522
- DE-U1- 20 308 672
- DE-U1- 20 312 113
- DE-U1- 20 318 351

## Beschreibung

Die Erfindung betrifft einen Stent gemäß den Merkmalen im Oberbegriff von Patentanspruch 1.

Stents dienen zur permanenten oder auch nur zeitweisen Schienung von Körperröhren, die infolge einer Stenose verschlossen oder verengt sind.

Die Stents weisen ein tubuläres Stützgerüst aus Metall auf, welches aus mehreren Ringsegmenten besteht. Diese sind aus sich über Übergangsabschnitte endlos aneinander schließenden Segmentstreben gebildet. In Längsachse des Stents benachbarte Ringsegmente sind durch Verbinderstreben gekoppelt.

Die Stents werden durch Kathetertechniken und ähnliche Einführhilfen in das intrakorporale Gefäß im Bereich der Stenose eingebracht und dort abgesetzt, wobei das Stützgerüst von einem gekrimpten Einbauzustand in einen demgegenüber im Durchmesser vergrößerten Stützzustand aufweitbar ist. Dieser Aufweitvorgang kann selbsttätig bei sogenannten selbst expandierenden Stents erfolgen, er kann aber auch mit Hilfe eines geeigneten Werkzeugs, beispielsweise eines Ballonkatheters herbeigeführt werden. Im Gefäß fungieren die Stents als Gefäßprothese zur Abstützung der Gefäßinnenwände.

In vielen Anwendungsfällen, beispielsweise in Gällengängen als sogenannter Biliary-Stent, muß der Stent nach einer relativ kurzen Verweildauer in der Körperröhre von wenigen Monaten wieder entfernt werden. Dies geschieht üblicherweise, indem man den Stent in einen Katheter zurückzieht. Der Durchmesser des aufgeweiteten und unter Umständen mit Körpersekret oder ähnlichem belegte Stent muß dabei wieder eingeschnürt werden. Für diesen Vorgang ist es erstrebenswert, dass sich von der Stentgeometrie nichts dagegen sperrt in einen im Durchmesser kleineren Katheter zurückgezogen zu werden.

Die DE 203 12 113 U1 offenbart einen Stent mit tubulärem Stützgerüst, bei dem jeweils benachbarte Ringsegmente in Axialrichtung über eine Verbinderstrebe gekoppelt sind. Die Verbinderstreben selbst weisen dabei einen sich in Umfangsrichtung erstreckenden U-förmigen Ausgleichsabschnitt auf.

Weiterhin ist aus der DE 203 18 351 U1 ein Stent bekannt, welcher ebenfalls in Längsrichtung benachbarte Ringsegmente aufweist. Die Ringsegmente weisen an einem Ende jeweils einander benachbarte Ringsegmente auf, die über eine Verbinderstrebe mit U- oder V-förmigen Ausgleichsabschnitten gekoppelt sind.

Der Erfindung liegt daher die Aufgabe zugrunde, einen Stent bereit zu stellen, der bei guter Gefäßverträglich im Stützzustand eine ausreichend hohe Flexibilität besitzt und der sich zum Entfernen aus einer Körperröhre gut krimpen lässt.

Die Lösung dieser Aufgabe besteht nach der Erfindung in einem Stent gemäß den Merkmalen von Patentanspruch 1.

Der erfindungsgemäße Stent weist ein tubuläres Stützgerüst auf, welches von einem Eingangszustand in einen Stützzustand aufweitbar ist. Das Stützgerüst besteht aus in Stentlängsachse aufeinander folgenden Ringsegmenten, die aus sich in Umfangsrichtung des Stützgerüstes endlos aneinander schließenden Segmentstreben gebildet sind. Benachbarte Ringsegmente sind durch Verbinderstreben gekoppelt. Kernpunkt der Erfindung bildet die Maßnahme, dass die Segmentstreben wellenförmig gekrümmt sind und dass jeder stirnseitige Übergangsabschnitt an zumindest einem in Stentlängsachse gesehen endseitigen Ringsegment ein axial vorstehendes verbreitertes Kopfende aufweist, wobei in Umfangsrichtung benachbarte Kopfenden in Stentlängsachse zueinander versetzt angeordnet sind und über Verbinderstreben zum benachbarten Ringsegment angeschlossen sind. Des Weiteren weist jede Verbinderstrebe einen wellenförmig gekrümmten Axialabschnitt und einen in Umfangsrichtung des Stützgerüstes weisenden V-förmig konfigurierten Ausgleichsabschnitt aufweist.

Vorteilhafte Ausgestaltungen und Weiterbildungen des erfindungsgemäßen Stents sind in den abhängigen Patentansprüchen 2 bis 8 charakterisiert.

Beim erfindungsgemäßen Stent sind zumindest an einem Ende alle Übergangsabschnitte der Segmentstreben jeweils über eine flexibel geformte Verbinderstrebe an das nächste benachbarte Ringsegment angebunden. Zum Entfernen kann der Stent an diesem Ende ergriffen und in einen im Durchmesser kleineren Katheter gezogen werden. Durch die erfindungsgemäße Design ist eine problemlose Einschnürung des Stützgerüstes möglich, ohne das nach außen ab- oder vorstehende Abschnitte des Stützgerüstes den Rückholvorgang sperren. Der Stent gleitet quasi unter einer gleichmäßigen Einschnürungsbewegung in den Katheter. Selbst verklebte oder verschleimte Stent lassen sich problemlos in einen Katheter zurückholen.

Die gerundeten Kopfenden gewährleisten einen schonenden Kontakt der stirnseitigen Enden des Stents an der Gefäßwand. Beim erfindungsgemäßen Stent werden die Gefäßwände demzufolge sowohl beim Setzen als auch beim Entfernen eines Stents weniger traumatisiert.

Das Stützgerüst weist ein Wellendesign auf ohne parallel zueinander verlaufenden gradlinige Strebenabschnitte, wobei sich die Segmentstreben einem kontinuierlichen Konturverlauf folgend vom mittleren Bereich jeweils zu ihren Enden hin verbreitern können. Diese Formgebung der Segmentstreben führt zu einem gleichmäßig verteilten Spannungsverlauf in den Segmentstreben.

Der Stent ist aus Metall gefertigt. Hierbei können alle verformbaren medizinisch möglichen Metalle bzw. Metalllegierungen zum Einsatz gelangen, z.B. Edelstahl, Kobaltlegierungen (Phynox), Reineisen oder insbesondere Nickel-Titan-Legierungen (Nitinol).

Für die Praxis interessant kann der erfindungsgemäße Stent auch als Kunststoff-Stent sein. Hierbei ist insbesondere die Verwendung von bioresorbierbaren Kunststoffen geplant. Vorzugsweise wird der Stent dann als Spritzformteil ausgeführt.

Die Flexibilität und Einschnürbarkeit des Stützgerüstes wird vorteilhaft unterstützt, wenn die V-förmig konfigurierten Ausgleichsabschnitte jeweils zwei in parallele Endabschnitte konvergierend auslaufende Schenkel aufweisen und die geneigten Längenabschnitte der Schenkel zudem einen gekrümmten Längenverlauf besitzen. Hierbei weisen die Ausgleichsabschnitte mit ihrem geschlossenen Ende alle in die selbe Umfangsrichtung des Stützgerüstes. Zudem sind die Ausgleichsabschnitte unmittelbar an die Übergangsabschnitte eines benachbarten Ringsegemnts angeschlossen.

Die Breite der Verbinderstreben, insbesondere die Breite der Axialabschnitte in den Verbinderstreben ist vorzugsweise geringer als die Breite der Segmentstreben der Ringsegmente.

Eine die Anwendung des erfindungsgemäßen Stents verbessernde Maßnahme besteht darin, dass die Übergangsabschnitte an dem in Stentlängsachse gesehen endseitigen Ringsegment ein axial vorstehendes verbreitertes Kopfende aufweisen, wobei benachbarte Kopfabschnitte in Stentlängsachse zueinander versetzt angeordnet sind. Jedes zweite Kopfende ist über einen Koppelabschnitt mit einem Übergangsabschnitt verbunden, wobei der Koppelabschnitt konkav gerundete Seitenkehlen aufweist. Die Seitenkehlen umgreifen im Einbauzustand des Stützgerüstes die benachbarten Kopfenden bereichsweise und decken diese ab.

Die vorteilhafterweise gerundeten Kopfenden gewährleisten einen schonenden Kontakt der stirnseitigen Enden des Stents an einer Gefäßwand. Hierdurch werden die Gefäßwände sowohl beim Setzen als auch beim Entfernen eines Stents weniger traumatisiert. Im gekrimpten Zustand decken die Kopfenden die benachbarten Übergangsabschnitte ab. Die Gefahr einer Verletzung der umliegenden Gefäßwände wird hierdurch deutlich verringert.

Die Funktionalität der Kopfenden kann weiter verbessert werden, wenn diese ösenartig gestaltet und mit einer Ausnehmung versehen sind. So können die Kopfenden genutzt werden, um den Stent mit einem geeigneten Werkzeug zu ergreifen oder aber, um einen Faden durch die Kopfenden hindurch zu fädeln bzw. schlingen. Vorzugsweise werden die Fadenenden in das Innere des Stützgerüstes umgelenkt und dort durch einen Verbinder, vorzugsweise aus einem röntgensichtbaren Material mit einander verbunden. Zum Entfernen des Stents können die Fadenenden am Verbinder ergriffen werden. Durch Ziehen wird der Faden eingeschnürt und hierbei das umschlungene Ringsegment des Stützgerüstes zusammengezogen, worauf der Stent aus der Körperröhre entfernt werden kann. Diese Vorgehensweise erleichtert wesentlich den Explantationsvorgang eines Stents.

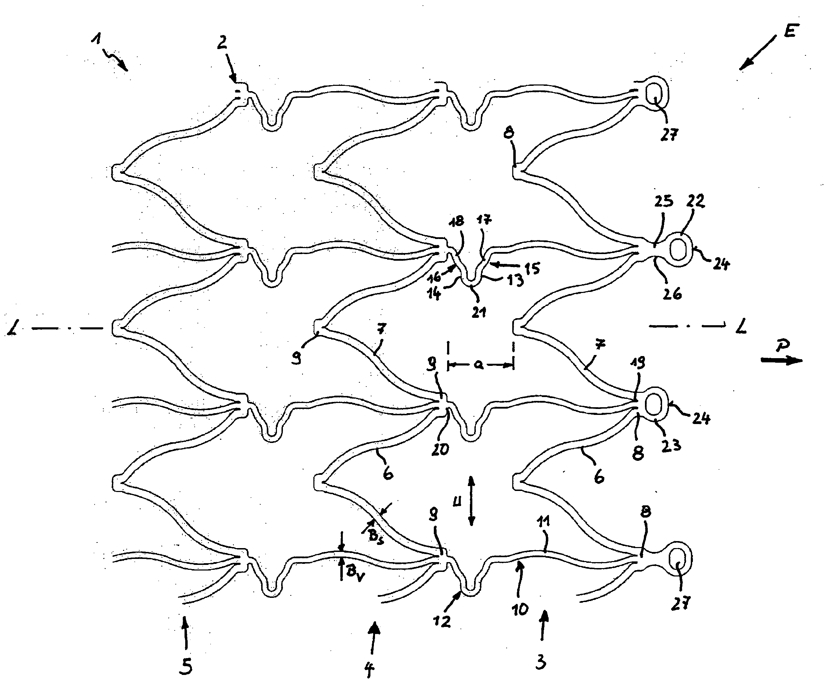

Die Erfindung ist nachfolgend anhand eines in der Zeichnung dargestellten Ausführungsbeispiels näher beschrieben. Die Zeichnung zeigt ein Ende E eines erfindungsgemäßen Stents 1 in einer Abwicklung im aufgeweiteten Stützzustand.

Der Stent 1 ist aus Metall gefertigt und besitzt ein tubuläres Stützgerüst 2 aus mehreren hintereinander folgenden Ringsegmenten 3, 4, 5. Die Länge eines Stents 1 kann grundsätzlich unterschiedlich sein. Wie erwähnt ist nur ein endseitiger Ausschnitt dargestellt und nicht die gesamte Anzahl der Ringsegmente 3, 4, 5 des Stents 1.

Die Ringsegmente 3, 4, 5 werden von zur Längsachse L des Stents 1 schräg verlaufenden und wellenförmig gekrümmten Segmentstreben 6, 7 gebildet, die sich über Übergangsabschnitte 8, 9 zick-zack-förmig endlos aneinander schließen. Untereinander sind die Ringsegmente 3, 4, 5 durch Verbinderstreben 10 gekoppelt. Man erkennt, dass jeder Übergangsabschnitt 8 des in Stentlängsachse L endseitigen Ringsegments 3 über eine Verbinderstrebe 10 mit dem Übergangsabschnitt 9 des benachbarten Ringsegments 4 verbunden ist. Jede Verbinderstrebe 10 weist einen wellenförmig gekrümmten Axialabschnitt 11 und einen in Umfangsrichtung U des Stützgerüstes weisenden V-förmig konfigurierten Ausgleichsabschnitt 12 auf. Hierbei sind die Ausgleichsabschnitte 12 jeweils in dem Raum zwischen den mit axialem Abstand a benachbarten Ringsegmenten 3, 4 bzw. 4, 5 angeordnet. Die Breite B_{A} der Axialabschnitte 11 ist dünner bemessen als die Breite B_{S} der Segmentstreben 6,7. Die Ausgleichsabschnitte 12 weisen jeweils zwei in parallele Endabschnitte 13, 14 konvergierend auslaufende Schenkel 15, 16 auf. Die geneigten Längenabschnitte 17, 18 der Schenkel 15, 16 haben einen gekrümmten Längenverlauf.

Die Verbinderstreben 10 erstrecken sich jeweils vom Übergangsabschnitt 8 im Tiefsten 19 zweier aneinander geschlossener Segmentstreben 6, 7 eines Ringsegments 3 bzw. 4 bis zum Übergangsabschnitt 9 an der Spitze 20 zweier aneinander geschlossener Segmentstreben 6, 7 des benachbarten Ringsegments 4 bzw. 5, wobei die Ausgleichsabschnitte 12 unmittelbar an die Übergangsabschnitte 9 angeschlossen sind. Hierbei weisen die Ausgleichsabschnitte 12 mit ihren geschlossenen Enden 21 alle in die selbe Umfangsrichtung U.

Die stirnseitigen Übergangsabschnitte 8 am endseitigen Ringsegment 3 weisen je ein axial vorstehendes verbreitertes Kopfende 22, 23 mit einem konkav gerundeten Stirnabschnitt 24 auf. In Umfangsrichtung U benachbarte Kopfenden 22, 23 sind zueinander versetzt angeordnet. Hierzu sind die Kopfenden 22 über einen Koppelabschnitt 25 mit den Übergangsabschnitten 8 verbunden, so dass die Kopfenden 22 gegenüber den Kopfenden 23 axial vorstehen.

Die Koppelabschnitte 25 weisen konkav gerundete Seitenkehlen 26 auf. Im gekrimpten Einbauzustand des Stützgerüstes 2 umgreifen die Kopfenden 22 die benachbarten Kopfenden 23 bereichweise und decken diese ab. Die Kopfenden 23 liegen so geschützt bzw. verdeckt durch die Kopfenden 22. Hierdurch werden die Gefäßwände sowohl beim Setzen als auch beim Entfernen eines Stents 1 weniger traumatisiert. Zudem gewährleisten die gerundeten Kopfenden 22, 23 eine behutsame Anlage des Stents 1 an der Gefäßwand beim Setzen.

Man erkennt ferner, dass die Kopfenden 22, 23 ösenartig gestaltet sind und Ausnehmungen 27 aufweisen. An den Ausnehmungen 27 kann der Stent 1 zum Entfernen aus einer Körperröhre ergriffen und in einen Katheter in Richtung des Pfeiles P gezogen werden. Hierbei kann das proximale Ende E auch zusätzlich eingeschnürt werden, beispielsweise mit Hilfe eines durch die Ausnehmungen 27 geschlungenen Fadens.

Von besonderem Vorteil für die Explantation eines Stents 1 ist die erfindungsgemäße Design des Stützgerüstes 2 mit den wellenförmigem Segmentstreben 6, 7 und die Konfiguration der Verbinderstreben 10 mit dem wellenförmigen Axialabschnitt 11 und dem Ausgleichsabschnitt 12, wobei die Axialabschnitte 12 dünner sind als die Segmentstreben 6, 7. Dies unterstützt die Flexibilität und Gleiteigenschaften des Stents 1. Erfindungwesentlich ist ferner, dass alle Übergangsabschnitte 8 am freien Ende E des Stents 1 bzw. des Ringsegments 3 jeweils über eine Verbinderstrebe 10 mit dem benachbarten Ringsegment 4 verbunden sind. Dies führt dazu, dass sich der Stent 1 zusammenziehen und in Richtung des Pfeiles P ziehen lässt, ohne dass sich von der Stentgeometrie etwas gegen den Explantationsvorgang sperrt. Der Stent 1 kann so ergriffen und in Richtung des Pfeiles P aus einer Körperröhre entfernt werden.

### Bezugszeichenaufstellung

- 1 -: Stent
- 2 -: Stützgerüst
- 3 -: Ringsegment
- 4 -: Ringsegment
- 5 -: Ringsegment
- 6 -: Segmentstrebe
- 7 -: Segmentstrebe
- 8 -: Übergangsabschnitt
- 9 -: Übergangsabschnit
- 10 -: Verbinderstrebe
- 11 -: Axialabschnitt
- 12 -: Ausgleichsabschnitt
- 13 -: Endabschnitt
- 14 -: Endabschnitt
- 15 -: Schenkel
- 16 -: Schenkel
- 17 -: Längenabschnitt
- 18 -: Längenabschnitt
- 19 -: Tiefste
- 20 -: Spitze
- 21 -: Ende v. 12
- 22 -: Kopfende
- 23 -: Kopfende
- 24 -: Stirnabschnitt
- 25 -: Koppelabschnitt
- 26 -: Seitenkehle
- 27 -: Ausnehmung
- L -: Stentlängsachse
- U -: Umfangsrichtung
- a -: Abstand
- E -: Ende v. 1
- P -: Pfeil
- B_{A} -: Breite v. 11
- B_{S} -: Breite v. 6,7

## Patentansprüche

1. Stent mit einem tubulären Stützgerüst (2) aus axial aufeinander folgenden Ringsegmenten (3, 4, 5), die aus sich über Übergangsabschnitte (8, 9) endlos aneinander schließenden Segmentstreben (6, 7) gebildet und benachbarte Ringsegmente (3, 4, 5) durch Verbinderstreben (10) gekoppelt sind, **dadurch gekennzeichnet, dass** die Segmentstreben (6, 7) wellenförmig gekrümmt sind und dass jeder stirnseitige Übergangsabschnitt (8) an zumindest einem in Stentlängsachse (L) gesehen endseitigen Ringsegment (3) ein axial vorstehendes verbreitertes Kopfende (22, 23) aufweist, wobei in Umfangsrichtung (U) benachbarte Kopfenden (22, 23) in Stentlängsachse (L) zueinander versetzt angeordnet sind und über Verbinderstreben (10) zum benachbarten Ringsegment (4) angeschlossen sind, wobei jede Verbinderstrebe (10) einen wellenförmig gekrümmten Axialabschnitt (11) und einen in Umfangsrichtung (U) des Stützgerüstes (2) weisenden V-förmig konfigurierten Ausgleichsabschnitt (12) aufweist.

2. Stent nach Anspruch 1, **dadurch gekennzeichnet, dass** die Ausgleichsabschnitte (12) jeweils zwei in parallele Endabschnitte (13, 14) auslaufende Schenkel (15, 16) aufweisen.

3. Stent nach Anspruch 2, **dadurch gekennzeichnet, dass** die geneigten Längenabschnitte (17, 18) der Schenkel (15, 16) einen gekrümmten Längenverlauf aufweisen.

4. Stent nach einem der Ansprüche 1 bis 3, **dadurch gekennzeichnet, dass** die Breite (B_{A}) der Axialabschnitte (11) dünner bemessen ist als die Breite (B_{S}) der Segmentstreben (6, 7).

5. Stent nach einem der Ansprüche 1 bis 4, **dadurch gekennzeichnet, dass** die Kopfenden (22, 23) ösenartig ausgebildet sind.

6. Stent nach einem der Ansprüche 1 bis 5, **dadurch gekennzeichnet, dass** jedes zweite Kopfende (22) über einen Koppelabschnitt (25) mit einem Übergangsabschnitt (8) verbunden ist, wobei der Koppelabschnitt (25) konkav gerundete Seitenkehlen (26) aufweist und die Seitenkehlen (26) im Einbauzustand des Stützgerüstes (2) die benachbarten Kopfenden (23) bereichsweise umgreifen.

7. Stent nach einem der Ansprüche 1 bis 6, **dadurch gekennzeichnet, dass** die Ausgleichsabschnitte (12) mit ihren geschlossenen Enden (21) in die selbe Umfangsrichtung (U) weisen.

8. Stent nach einem der Ansprüche 1 bis 7, **dadurch gekennzeichnet, dass** die Ausgleichsabschnitte (12) unmittelbar an die Übergangsabschnitte (9) des benachbarten Ringsegments (4, 5) angeschlossen sind.

## Claims

1. Stent with a tubular support frame (2) made of axially sequential ring segments (3, 4, 5) which are formed by segmented braces (6, 7) interconnected continuously via transfer sections (8, 9) and adjacent ring segments (3, 4, 5) are coupled by connecting braces (10), **characterised in that** the segmented braces (6, 7) are curved in a wavelike manner and that each front end transfer section (8) has an axially projecting widened head end (22, 23) on at least one back end ring segment (3) viewed in the longitudinal axis (L) of the stent wherein adjacent head ends (22, 23) are arranged offset in the circumferential direction (U) in the longitudinal axis of the stent (L) and are connected to the adjacent ring segment (4) via connecting braces (10), wherein each connecting brace (10) has an axial section (11) curved in a wavelike manner and a compensation segment (12) configured in a V-shape pointing in the circumferential direction (U) of the support frame (2).

2. Stent according to claim 1, **characterised in that** the compensation segments (12) respectively have two limbs (15, 16) running in parallel end sections (13, 14).

3. Stent according to claim 2, **characterised in that** the inclined longitudinal sections (17, 18) of the limbs (15, 16) have a curved longitudinal profile.

4. Stent according to any one of claims 1 to 3, **characterised in that** the width (B_{A}) of the axial sections (11) is dimensioned thinner than the width (B_{S}) of the segmented braces (6, 7).

5. Stent according to any one of claims 1 to 4, **characterised in that** the head ends (22, 23) are designed in the shape of a loop.

6. Stent according to any one of claims 1 to 5, **characterised in that** each second head end (22) is connected to a transfer section (8) via a coupling section (25) wherein the coupling section (25) has side grooves (26) rounded in a concave manner and the side grooves (26) encompass the adjacent head ends (23) in regions in the installed state of the support frame (2).

7. Stent according to any one of claims 1 to 6, **characterised in that** the compensation sections (12) point with their closed ends (21) in the same circumferential direction (U).

8. Stent according to any one of claims 1 to 7, **characterised in that** the compensation sections (12) are connected directly to the transfer sections (9) of the adjacent ring segments (4, 5).

## Revendications

1. Stent avec un treillis de soutien tubulaire (2) composé de segments annulaires (3, 4, 5) qui se suivent les uns les autres de façon axiale et qui sont formés de jambes de segment (6, 7) se raccordant sans fin les unes aux autres par l'intermédiaire de tronçons de transition (8, 9), des segments annulaires (3, 4, 5) voisins étant couplés par des jambes de liaison (10), **caractérisé en ce que** les jambes de segment (6, 7) sont courbées en forme d'ondes et **en ce que** chaque tronçon de transition (8) frontal comporte au niveau d'au moins un segment annulaire (3) d'extrémité vu dans l'axe longitudinal de stent (L) une tête (22, 23) élargie et dépassant de façon axiale, des têtes (22, 23) voisines en direction circonférentielle (U) étant disposées décalées les unes par rapport aux autres dans la direction de l'axe longitudinal de stent (L) et étant raccordées par l'intermédiaire de jambes de liaison (10) au segment annulaire (4) voisin, chaque jambe de liaison (10) ayant un tronçon axial (11) courbé en forme d'onde et un tronçon de compensation (12) configuré en forme de V dirigé dans la direction circonférentielle (U) du treillis de soutien (2).

2. Stent selon la revendication 1, **caractérisé en ce que** les tronçons de compensation (12) ont chacun deux branches (15, 16) s'étendant en tronçons terminaux (13, 14) parallèles.

3. Stent selon la revendication 2, **caractérisé en ce que** les tronçons longitudinaux (17, 18) inclinés des branches (15, 16) ont une extension longitudinale courbé.

4. Stent selon l'une des revendications 1 à 3, **caractérisé en ce que** la largeur (B_{A}) des tronçons axiaux (11) est plus mince que la largeur (B_{S}) des jambes de segment (6, 7).

5. Stent selon l'une des revendications 1 à 4, **caractérisé en ce que** les têtes (22, 23) sont réalisées en forme d'oeillets.

6. Stent selon l'une des revendications 1 à 5, **caractérisé en ce que** chaque deuxième tête (22) est reliée par l'intermédiaire d'un tronçon de couplage (25) à un tronçon de transition (8), le tronçon de couplage (25) ayant des gorges latérales (26) arrondies concaves et les gorges latérales (26) entourant par endroits les têtes voisines (23) lorsque le treillis de soutien (2) est installé.

7. Stent selon l'une des revendications 1 à 6, **caractérisé en ce que** les tronçons de compensation (12) pointent avec leurs extrémités fermées (21) dans la même direction circonférentielle (U).

8. Stent selon l'une des revendications 1 à 7, **caractérisé en ce que** les tronçons de compensation (12) sont raccordés directement aux tronçons de transition (9) du segment annulaire (4, 5) voisin.
